# EUROPEAN PATENT SPECIFICATION

(11) **EP 1 632 559 B1**
(45) Date of publication and mention of the grant of the patent: **22.08.2007**
(21) Application number: 05291663.2
(22) Date of filing: 04.08.2005
(51) Int. Cl.: C11D 3/50, C11D 3/20, C11D 7/26, A61L 9/01

(54) **Household products, including inside air-care products.**
Wasch- und Reinigungsmittel, sowie Raumsprays
Produits ménagers, incluant les désodorisants d'intérieur

(30) Priority: 06.08.2004 FR 0408727; 22.12.2004 FR 0413710
(43) Date of publication of application: 08.03.2006
(73) Proprietor: Takasago International Corporation, Tokyo 144-8721 (JP)
(72) Inventor: Warr, Jonathan, 12, rue Torricelli, 75017 Paris (FR); Unno, Masakatsu, 12, rue Torricelli, 75017 Paris (FR); Regniez, Catherine, 12, rue Torricelli, 75017 Paris (FR)
(74) Representative: Gillard, Marie-Louise

(56) References cited:
- WO-A-98/06803
- WO-A-02/089862
- GB-A- 1 000 783
- US-A- 5 800 897
- US-A- 6 025 406
- US-A- 6 054 137
- US-A1- 2004 101 459
- US-B1- 6 177 070
- US-B1- 6 194 362

## Description

The present invention relates to household products and in particular to perfumes used in household products.

In the present description, by "household products", reference is made to products used for cleaning, polishing and disinfecting any type of surface in an area, such as a house, office, shop etc. as well as products for the treatment of clothes, sheets etc., such as washing powders and detergents, softeners etc. as well as inside deodorants which diffuse perfumes generating a particular aesthetic atmosphere (ambiance) and which are referred to as "air-care products", such as for example candles, gels, membrane air care devices and electrically powered air-care devices optionally provided with a fan.

By "inside", reference is made in the present description to a limited space, such as for example, the inside of a dwelling or a vehicle.

It is normal practice to add perfumes to household products in order to make housework more pleasant or to procure a pleasant sensation for users of surfaces treated with household products or of clothes, sheets etc. cleaned with such products. Furthermore, air care products are commonly used to perfume the surrounding air.

The manufacturers of household products are faced with the need to provide consumers with products that not only show excellent cleaning performance but also give rise to a sensation of well-being and produce psychological effects in consumers, such as stimulant and/or sedative effects.

As a perfume having a sedative effect, an essential oil can be used, such as for example the essential oil of lavender, the essential oil of bergamot, the essential oil of lemon, the essential oil of marjoram, or the essential oil of sandalwood (M.Indo, Koryo No 168, 43 (1990).

It is very important that the odoriferous (odour-providing) properties of the perfume are stable over time.

In effect, household products are not used immediately after their manufacture. Indeed, there are often consumed a few months after manufacture.

It has been found that the essential oils mentioned above lose their odoriferous properties in household products over a period of time and therefore their use in household products is of uncertain value.

Although such oils may have a satisfactory effect when the household products are used immediately after their manufacture, their odoriferous properties fade away over the course of time and sometimes the oils are even transformed with the production of an undesirable odour.

With air care products, in particular with electrically powered air care devices, diffusion is carried out in a uniform manner during a certain period of time, for example 30 to 45 days, and the ambiance-generating perfume must maintain its odoriferous note without change.

Essential oils, in particular the essential oil of lemon, are not adapted to these air care devices and an irregular loss of weight over time is observed as well as a modification as regards the odoriferous note.

With perfumed candles, the incorporation of essential oils is a difficult procedure. In effect, at the temperature of molten wax, the volatile components of the essential oils can easily evaporate, which changes the odoriferous note required. Furthermore, such candles produce a large amount of soot, which is not acceptable for the consumer.

The applicant has therefore sought to develop perfumes that can be used in household products and which maintain their odoriferous properties over a period of time.

The applicant has now found, quite surprisingly, that alkoxybenzenes of the formula (I) hereunder can be used in household products for their odoriferous properties.

In effect, it has been found that these derivatives are particularly stable in household products and that they can be easily incorporated into all types of household products, including inside air care products, and in particular candles.

Furthermore, these compounds have a sedative effect which is of particular interest at the present time in view of the stress arising from daily life which leads to various symptoms, including insomnia.

In the present application, "sedative effect" refers to psychological aspects of well-being and encompasses calming, soothing, and relaxing effects, as well as effects of stress reduction or elimination etc.

The invention therefore has as its object the use of at least one alkoxybenzene of formula (I) : 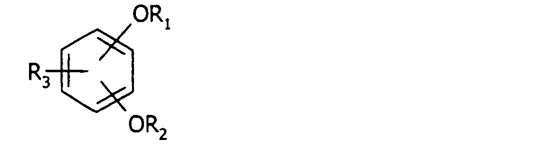 in which :
- R₁ and R₂, which can be the same or different, are a (C₁-C₄) alkyl group, preferably methyl or ethyl;
- R₃ is a (C₁-C₄) alkyl group or a group of formula -OR₄, R₄ being a (C₁-C₄) alkyl group;
as an odoriferous agent for household products.

Preferably, R₁ and R₄ are the same.

The quantity of alkoxybenzene which is appropriate for carrying out the invention varies according to the nature of the household product. Thus, for household products other than air care products, the quantity of alkoxybenzene is generally comprised between 0.0001 and 0.1 % by weight of the overall composition of the household product.

For air care products, the quantity of alkoxybenzene is generally comprised between 0.01 and 15 % by weight of the overall composition of the deodorant.

The invention also has as its object perfume compositions for household products which contain 0.01 and 10 % of at least one alkoxybenzene of formula (I) as defined above.

In the compositions above, one or more than one alkoxybenzene of formula (I) may be used.

Among the alkoxybenzenes of formula (I) above, the most preferred are 1,3,5-trimethoxybenzene, 1,3-dimethoxy-5-methylbenzene and mixtures thereof.

The alkoxybenzenes which are suitable in order to carry out the invention are known products and are available from commercial sources.

They can easily be manufactured by the methods described or referred to by Liu et al, Organic Preparations and Procedures International, Vol 35 (2003), 223.

The perfume compositions which can be used in carrying out the invention are compositions commonly used in the field of household products.

Examples of suitable perfume compositions are described in particular in patents US 6 743 768 and GB 2 355 015.

Alkoxybenzenes of formula (I) can be used in all sorts of household products, be they liquid or solid.

Concerning household products, reference may be made to the following works:
- Surfactant Science Series, Vol 71, Powdered Detergent ISBN 0-8247-9988-7,
- Surfactant Science Series Vol 67 Liquid Detergents ISBN 0-8247-9391-9 (Marcel Dekker Inc),
as well as to patents or patent applications US 2003/0134769 A1; WO 2004/041983 A1 ; EP 0 918 835 A1 and WO 2003/022972 A1.

The stability of alkoxybenzenes and of essential oils in household products was determined by the test described in the illustrative examples.

The sedative effect of an alkoxybenzene according to the invention was determined by measuring the variations of negative electrical potential, called "contingent negative variations" (CNV) according to the method of Torii [Proceedings of the 19^{th} Japanese Symposium on Taste and Smell, 65 (1985)], which is also described in detail in patent US 6 268 333.

Contingent negative variations are small variations in cerebral potential related to psychological processes, such as attention, waiting, anticipation etc. as well as being related to changes in the state of consciousness.

According to this method, a luminous signal is emitted approximately 2 seconds after an audible signal, and the subject must switch off the luminous signal by pressing on the button as soon as the luminous signal is recognised.

When carrying out these experiments, a sample to be tested (perfume composition) or a reference sample (with no smell) is placed around 10 centimetres from the nose of the subject taking part in these experiments in such a way as to ensure that the compound is recognised at all times via the said subject's respiration.

An electrode for measuring the contingent negative variations is placed on the subject's forehead (the reference electrode is placed on the ear lobe).

The contingent negative variations measured during an early period of time going from 400 msec to 1000 msec after the audible signal and the amplitude of these variations is expressed with respect to a reference sample to which a value of 100% is given.

A sample tested whose amplitude is greater than 100% will be a stimulant product and a sample tested with an amplitude less than 100% will be a sedative.

The measurement of contingent negative variations has shown that 1,3-dimethoxy-5-methylbenzene, trimethoxybenzene, and in particular, 1,3,5-trimethoxybenzene, had a sedative effect when used in household products comprised between 0.0001 % and 0.1 % by weight with respect to the total weight of the said household product.

The invention will now be described in more detail by the illustrative and non-limiting examples hereunder in which the following abbreviations have been used:
Na-LAS = sodium (linear) alkyl benzene sulfonate
Na-PAS = sodium (linear) alkyl sulfate
Na-AES = sodium alkyl ether sulfate
SCMC = sodium carboxymethylcellulose
TAED = tetraacetylethylamine
APG = alkylpolyglucoside

### EXAMPLES 1 to 7

The household products containing the ingredients mentioned in tables 1 to 7 hereunder were prepared using mixing techniques well known to the person skilled in the art.

### EXAMPLE 1 : Perfume composition (perfume n°1) for powder detergent.

| Ingredient | Chemical name | CAS N° | Parts per 1000 |
|---|---|---|---|
| AMBROXAN (AMBROFIX) | Dodecahydro-3a,6,6,9a-tetramethylnaphtho (2,1-b) furan | 6790-58-5 | 15 |
| LEVOSANDOL | 2-ethyl-4-(2,2,3-trimethyl-3-cyclopenten-1-yl)-2-buten-1-ol | 28219-61-6 | 10 |
| L-CITRONELLOL | 3, 7-dimethyl-6-octen-1-ol | 7540-51-4 | 15 |
| COUMARIN | 1,2-benzopyrone | 91-64-5 | 5 |
| CYCLACET | Tricyclodecenyl acetate | 5413-60-5 | 30 |
| CYCLAPROP | Tricyclodecenyl propionate | 17511-60-3 | 30 |
| DIHYDRO MYRCENOL | 2,6-dimethyl-7-octen-2-ol | 18479-58-8 | 60 |
| DIPROPYLENE GLYCOL | | 25265-71-8 | 70 |
| DIMETHYL BENZYL CARBINYL ACETATE | Alpha-alpha-dimethyl-phenylethyl acetate | 151-05-3 | 15 |
| EXALTOLIDE TOTAL | Omega-pentadecalactone | 106-02-5 | 75 |
| GERANYL NITRILE | 3,7-dimethyl-2,6-octadiene-1-nitrite | 5146-66-7 | 5 |
| HEDIONE | Methyl dihydrojasmonate | 24851-98-7 | 115 |
| BETA IONONE | 4-(2,6,6-trimethyl-1-cyclohexen-1-yl)-3-buten-2-one | 14901-07-6 | 15 |
| ORBITONE | Octahydro-2,3,8,8-tetramethyl-2-acetonaphtone | 54464-57-2 | 340 |
| LINALOOL | 3,7-dimethyl-1,6-octadien-3-ol | 78-70-6 | 35 |
| MAYOL | Cis-4-(1-methylethyl)cyclohexanemethanol | 13828-37-0 | 25 |
| METHYL IONONE PURE | | 127-43-5 | 80 |
| METHYL NAPTHYL KETONE CRYSTAL | | 93-08-3 | 10 |
| NEROLIN BROMELIA | Beta-naphthyl ethyl ether | 93-18-5 | 10 |
| STYRALLYL ACETATE | α-Methylbenzyl acetate | 93-92-5 | 10 |
| 1,3,5-TRIMETHOXYBENZENE | | 621-23-8 | 10 |
| VERDOX | o-t-Butylcyclohexyl acetate | 88-41-5 | 20 |

### EXAMPLE 2 : Perfume composition (perfume n°2) for powder detergent.

| Ingredient | Chemical name | CAS N° | Parts per 1000 |
|---|---|---|---|
| L-BORNEOL, CRYSTAL | Bornylic alcohol / 2-Hydroxycamphane | 507-70-0 | 5 |
| L-CITRONELLOL | | 5413-60-5 | 5 |
| CYCLACET | Tricylodecenyl acetate | | 25 |
| DIHYDRO MYRCENOL | 2,6-dimethyl-7-octen-2-ol | 18479-58-8 | 255 |
| 3,5-DIMETHOXYTOLUENE | | 4179-19-5 | 5 |
| DIPROPYLENE GLYCOL | | 25265-71-8 | 45 |
| KOAVONE | Acetyl-diisoamylene | 81786-73-4 | 30 |
| LINALOOL | 3,7-dimethyl-1,6-octadien-3-ol | 78-70-6 | 5 |
| LINALYL ACETATE | 3,7-dimethyl-1,6-octadien-3-yl acetate | 115-95-7 | 15 |
| NORLIMBANOL DEXTRO | Firmenich base | 70788-30-6 | 10 |
| ORBITONE | Octahydro-2,3,8,8-tetramethyl-2-acetonaphtone | 54464-57-2 | 475 |
| PHENYL ETHYL ALCOHOL | | 60-12-8 | 125 |

### EXAMPLE 3 : Perfume compositions (perfume n°3 and perfume n°4) for liquid washing composition.

| Ingredient | Chemical name | CAS N° | Perfume n° 3 parts per 1000 | Perfume n°4 parts per 1000 |
|---|---|---|---|---|
| ALDEHYDE C - 12 MNA | | 110-41-8 | 5 | 5 |
| GAMMA UNDECALACTONE | | 104-67-6 | 5 | 5 |
| AMBER CORE | 2-t-butylcyclohexyloxy-2-butanol | 139504-68-0 | 10 | 10 |
| AMYLESALICYLATE | | 2050-08-0 | 90 | 90 |
| APHERMATE | a,3,3-trimethylcyclohexylmethyl formate | 25225-08-5 | 100 | 100 |
| LEVOSANDOL | 2-ethyl-4-(2,2,3-trimethyl-3-cyclopenten-1-yl)-2-buten-1-ol | 28219-61-6 | 15 | 15 |
| CEDRAMBER | | 19870-74-7 | 5 | 5 |
| CITRALVA | 3,7-dimethyl-2,6-octadiene-1-nitrile | 31983-27-4 | 10 | 10 |
| L-CITRONELLOL | | 7540-51-4 | 90 | 90 |
| CYCLACET | Tricyclodecenyl acetate | 5413-60-5 | 40 | 40 |
| EUGENOL | | 97-53-0 | 10 | 10 |
| DIHYDRO MYRCENOL | 2,6-dimethyl-7-octen-2-ol | 18479-58-8 | 100 | 100 |
| 3,5-DIMETHOXYTOLUENE | | 4179-19-5 | | 10 |
| DIPROPYLENE GLYCOL | | 25265-71-8 | 94 | 99 |
| GALBEX 183 | GALBEX 183 (Firmenich base) | | 2 | 2 |
| CIS-3 HEXEN-1-OL | | 928-96-1 | 2 | 2 |
| HEXYL SALICYLATE | n-Hexyl-ortho-hydroxybenzoate | 6259-76-3 | 160 | 160 |
| ALPHA-IONONE | 4-(2,2,6-trimethyl-2-cyclohexen-1-yl)-3-buten-2-one | 127-41-3 | 15 | 15 |
| LINALOOL | 3,7-dimethyl-1,6-octadien-3-ol | 78-70-6 | 15 | 15 |
| ORBITONE | Octahydro-2,3,8,8-tetramethyl-2-acetonaphthone | 54464-57-2 | 45 | 45 |
| PHENYL ETHYL ALCOHOL | | 60-12-8 | 15 | 15 |
| PHENYLETHYL PHENYLACETATE | | 102-20-5 | 2 | 2 |
| ROSEPHENONE | | | 10 | 10 |
| 1,3,5-TRIMETHOXYBENZENE | | 621-23-8 | 15 | |
| VERDOX | o-t-Butylcyclohexyl acetate | 88-41-5 | 110 | 110 |
| VERTOFIX COEUR | Methyl Cedryl Ketone | 32388-55-9 | 35 | 35 |

### EXAMPLE 4 : Perfume compositions (perfumes n°5, 6 and 7) for liquid softener.

| Ingredient | Chemical name | CAS N° | Perfume n°5 parts per 1000 | Perfume n° 6 parts per 1000 | Perfume n° 7 parts per 1000 |
|---|---|---|---|---|---|
| GAMMA UNDECALACTONE | γ-undecalactone | 104-67-6 | 20 | 20 | 20 |
| ANISIC ALDEHYDE | p-methoxybenzaldehyde | 123-11-5 | 15 | 15 | 15 |
| BENZYL ACETATE | | 140-11-4 | 205 | 205 | 205 |
| BENZYL SALICYLATE | Benzyl-ortho-hydroxybenzoate | 118-58-1 | 15 | 15 | 15 |
| CITRONELLOL 950 | 3,7-dimethyl-6-octen-1-ol | 106-22-9 | 30 | 30 | 30 |
| COUMARIN | 1,2-benzopyrone | 91-64-5 | 20 | 20 | 20 |
| Para CRESYL METHYL ETHER | p-methyl anisole / p-methoxy toluene *p-*tolyl methyl ether | 104-93-8 | 1 | 1 | 1 |
| 3,5-DIMETHOXYTOLUENE | | 4179-19-5 | | 10 | 5 |
| DIPROPYLENE GLYCOL | Dipropylene glycol | 25265-71-8 | 94 | 89 | 89 |
| ETHYL VANILLIN | 3-ethoxy-4-hydroxybenzaldehyde | 121-32-4 | 60 | 60 | 60 |
| HEDIONE | Methyl dihydrojasmonate | 24851-98-7 | 170 | 170 | 170 |
| HELIOTROPIN | 3,4-methylene dioxybenzaldehyde | 120-57-0 | 15 | 15 | 15 |
| ALPHA-HEXYL-CINNAMALDEHYDE | | 101-86-0 | 15 | 15 | 15 |
| LILIAL | p-tert-butyl-alpha-methyldihydrocinnamic aldehyde | 80-54-6 | 30 | 30 | 30 |
| D-LIMONENE | | 5989-27-5 | 10 | 10 | 10 |
| LINALOOL | 3,7-dimethyl-1,6-octadien-3-ol | 78-70-6 | 100 | 100 | 100 |
| METHYL METHYL BENZOATE | | 95-58-3 | 5 | 5 | 5 |
| METHYL ISO EUGENOL | Isoeugenyl methyl ether | 93-16-3 | 5 | 5 | 5 |
| PHENYL ETHYL ALCOHOL | | 60-12-8 | 100 | 100 | 100 |
| TERPINEOL | Menth-1-en-8-ol | 98-55-5 | 50 | 50 | 50 |
| TERPINYL ACETATE | Menth-1-en-8-yl acetate | 80-26-1 | 20 | 20 | 20 |
| 1,3,5-TRIMETHOXYBENZENE | | 621-23-8 | 5 | | 5 |
| VERTOFIX COEUR | Methyl Cedryl Ketone | 32388-55-9 | 15 | 15 | 15 |

**EXAMPLE 5 : Standard powder detergent (standard powder detergents n°1 to n°3) or concentrated powder detergent (concentrated powder n°1).**

| | Standard powder n° 1 | Standard powder n° 2 | Standard powder n° 3 | Concentrated powder n°1 |
|---|---|---|---|---|
| | | | | |
| NA-LAS | 8.5 | 11 | 11 | 8 |
| NA-PAS | | | 3.5 | 5.5 |
| NA-AES | | | | 1.5 |
| NONIONIC 7EO | 6.5 | 3.5 | 3.5 | 5 |
| CATIONIC | | | | 1.3 |
| SOAP | 2 | 1 | 1.2 | 0.3 |
| ZEOLITE A24 | 19.5 | | | |
| ZEOLITE A4 | | 22 | 3.5 | 20 |
| COPOLYMER CP5 | 1.7 | 3 | | 1 |
| POLYACRYLATE (PM 5000) | | | 3.5 | |
| NA CITRATE / CITRIC ACID | 2.5 | | 1.5 | 4 |
| NA SILICATE | 1.5 | | | |
| NA DISILICATE (SKS-6) | | 2.5 | 3.5 | 11 |
| NA CARBONATE | 18.5 | 18.5 | 26.5 | 14 |
| NA SULPHATE | 27.5 | 10 | 21 | 4 |
| SCMC | 0.15 | | | 0.15 |
| TAED (83% MA) | 1 | 4 | 3.5 | 5 |
| NA PERCARBONATE | 7 | | 13 | 13 |
| NA PERBORATE | | 19 | | |
| | | | | |
| PERFUME 1 | 0.35 | | | 0.55 |
| PERFUME 2 | | 0.4 | 0.4 | |

### EXAMPLE 5 : Standard powder detergent (standard powder detergents n°1 to n°3) or concentrated powder detergent (concentrated powder n°1).

### (Continued)

| | Standard powder n° 1 | Standard powder n° 2 | Standard powder n° 3 | Concentrated powder n°1 |
|---|---|---|---|---|
| ANTI-FOAMING AGENT (15% MA) | 1 | 0.7 | 0.7 | 1 |
| FLUORESCENCE AGENT (15% MA) | 1 | 0.7 | 0.7 | 0.7 |
| DEFLOCULATING AND SEQUESTRATING AGENT (DEQUEST® 2047 AND 2016) | 0.8 | 0.8 | 1.5 | 1.5 |
| ENZYMES (PROTEASE, LIPASE, CELLULASE, AMYLASE) | 0.3 | 1 | 1 | 1.5 |
| COLOURED CARBONATE | | 1.5 | | |
| POLYMERS FOR FABRIC MAINTENANCE (ELIMINATION OF STAINS, DYE TRANSFER, ETC.) | 0.2 | 0.4 | 0.5 | 1 |
| | | | | |
| TOTAL | 100 | 100 | 100 | 100 |

### EXAMPLE 6 : Standard liquid detergent (St. Liq. n°1 to n°4) or concentrated liquid detergent (Conc. Liq. n°1 to n°4).

| | St Liq 1 | St Liq 2 | St Liq 3 | St Liq 4 | Conc Liq 1 | Conc Liq 2 | Conc Liq 3 | Conc Liq 4 |
|---|---|---|---|---|---|---|---|---|
| | | | | | | | | |
| NA-LAS | 9.5 | | | | 14 | | | |
| NA-PAS | | 4 | 10 | 7 | 4 | 5 | 5 | 15 |
| NA-AES | | | 2 | 2 | | | | 2.6 |
| NONIONIC 7EO | 15 | 9 | 3.5 | 4.5 | 15 | 24 | 22 | |
| CATIONIC | | | 1 | | | | | 2 |
| SOAP | 15 | 15 | 7 | 6 | 12 | 17 | 18 | 11 |
| APG | | 4 | | | | 4 | 2.5 | |
| GLUCOSAMIDE | | | 4.5 | 4 | | | | 6 |
| MONO ETHANOL AMINE | | | 5 | 3.5 | | | | 6.5 |
| CITRATE | 1 | 1 | 2 | 1.2 | 6 | 1 | 4 | 2 |
| PROPYLENE GLYCOL | | 3 | 6 | 6 | 5 | | 3 | 8 |
| GLYCEROL | | | | | | 4 | | |
| ETHANOL/IMS (INDUSTRIAL ALCOHOLS) | 7 | 2 | 1 | 1 | | 7 | 1.5 | 2.2 |
| | | | | | | | | |
| PERFUME N° 3 | 0.4 | | 0.5 | | 0,6 | | 0.7 | |
| PERFUME N° 4 | | 0.4 | | 0.4 | | 0.7 | | 0.7 |
| | | | | | | | | |
| ANTI-FOAMING AGENT (15% MA) | 1 | 0.7 | 0.7 | 0.7 | 1 | 1 | 1 | 1 |
| FLUORESCENCE AGENT (15% MA) | 1.1 | 0.7 | 0.7 | 0.7 | 1 | 1 | 1 | 1 |
| DEFLOCULATING AND SEQUESTRATING AGENT (DEQUEST® 2040 AND 2010) | 1 | 1 | 1.4 | 1 | 1.5 | 1.5 | 1.5 | 1.5 |

### EXAMPLE 6 : Standard liquid detergent (St. Liq. n°1 to n°4) or concentrated liquid detergent (Conc. Liq. n°1 to n°4).

### (continued)

| | St Liq 1 | St Liq 2 | St Liq 3 | St Liq 4 | Conc Liq 1 | Conc Liq 2 | Conc Liq 3 | Conc Liq 4 |
|---|---|---|---|---|---|---|---|---|
| | | | | | | | | |
| ENZYMES (PROTEASE, LIPASE, CELLULASE, AMYLASE) | 0.7 | 1.05 | 1.05 | 0.9 | 1.2 | 1.2 | 1.2 | 1.2 |
| POLYMERS FOR FABRIC MAINTENANCE (ELIMINATION OF STAINS, DYE TRANSFER, ETC.) | 1 | 1 | 1 | 1 | 1.5 | 1.5 | 1.5 | 1.5 |
| WATER | 47.3 | 57.15 | 52.65 | 60.1 | 37.2 | 31.1 | 37.1 | 32.8 |
| | | | | | | | | |
| TOTAL | 100 | 100 | 100 | 100 | 100 | 100 | 100 | 100 |

### EXAMPLE 7 : Standard Softener (St FC 1) or concentrated liquid softener (Conc FC 1-2)

| | St FC 1 | | Conc FC 1 | Conc FC 2 |
|---|---|---|---|---|
| | | | | |
| TETRANYL AHT-1 | 5.0 | | 12.0 | |
| DEQA | | | | 18 |
| GENAPOL C200 | 0.1 | | 0.75 | 3 |
| ISOPROPYL ALCOHOL | | | | 3 |
| POLYETHYLENE GLYCOL 4000 | | | | 0.6 |
| LAUREX CS | 0.4 | | 1.8 | |
| | | | | |
| PERFUME N° 5 | 0.3 | | | |
| PERFUME N° 6 | | | 0.9 | |
| PERFUME N° 7 | | | | 0.7 |
| | | | | |
| DYE, ANTIFOAMING AGENT, PRESERVATIVE | qs | | qs | Qs |
| MAGNESIUM CHLORIDE | qs | | qs | Qs |
| | | | | |
| WATER QSP | 100 | | 100 | 100 |

| | | | | |
|---|---|---|---|---|
| Tetranyl AHT-1 ; semi-hard tallow ester of triethanolammonium methosulphate, marketed by Kao Corp Genapol C200 ; copra ethoxylate, marketed by Clariant Laurex CS ; Long chain alcohol, marketed by Albright & Wilson DEQA ; soft di(tallow oxyethyl) dimethylammonium chloride | | | | |

### EXAMPLE 8 : Determination of stability

0.15 % by weight of 1,3,5-trimethoxybenzene (TMB) or of 1,3-dimethoxy-5-methylbenzene (DMB) or of an essential oil were mixed in a commercial detergent preparation not containing any perfume known under the trade name "PERSIL SENSITIV" marketed in Germany by Henkel.

The essential components of this detergent are:

| | |
|---|---|
| Less than 5 % | Phosphonate, soap, nonionic surfactant, polycarboxylate |
| 15 à 30 % | Bleaching agent, anionic surfactant, zeolite (SASIL^{®}) |
| | Enzyme (Amylase, Cellulase, Protease) |
| | TAED, Soda, sodium citrate, optical brightener |

Samples of this detergent were stored in cardboard packaging sold by the company Gandolphe Emballages Sarl at 40°C with 70 % relative humidity.

Stability was judged by a perfume specialist according to the following scale of values 1-5:
1 - stable (no modification)
2 - stable (no modification, but loss of the leading note observed)
3 - acceptable (very slightly modified)
4 - borderline (slightly modified)
5 - unstable (modified and bad).

The essential oils known for their relaxing effects were stored as indicated above and their odoriferous properties were judged after five weeks of storage and compared to those of TMB or DMB stored in the same conditions.

The results gathered together in the table below clearly show that the essential oils are unstable whereas DMB and TMB are stable.

| Essential oils | stability |
|---|---|
| Essence of bergamot | 5 |
| Essence of camomile | 4 |
| Essence of lemon | 5 |
| Essence of marjoram | 4 |
| Essence of sandalwood | 4 |
| Essence of lavender | 5 |
| TMB (used in the form of a 25 % solution in dipropylene glycol) | 2 |
| DMB | 2 |

### EXAMPLE 9 : Perfume composition for electrically powered air care devices.

| Ingredient | Chemical name | CAS N° | Parts per 1000 |
|---|---|---|---|
| HEDIONE | Methyl dihydrojasmonate | 24851-98-7 | 120 |
| 1,3,5-TRIMETHOXYBENZENE | | 621-23-8 | 100 |
| ORBITONE | Octahydro-2,3,8,8-tetramethyl-2-acetonaphtone | 54464-57-2 | 100 |
| DI-HYDRO MYRCENOL | 2,6-dimethyl-7-octen-2-ol | 18479-58-8 | 40 |
| MUSK-T | 1,4-Dioxacycloheptadecane-5,17-dione | 105-95-3 | 40 |
| LEVOSANDOL | 2-ethyl-4-(2,2,3-trimethyl-3-cyclopenten-1-yl)-2-buten-1-ol | 28219-61-6 | 8 |
| CITRAL | 3,7-dimethyl-2,6-octadienal (mixture of cis and trans) | 5392-40-5 | 5 |
| ISO BUTYL QUINOLINE (1% IN DPG) | 6-(1-Methylpropyl)quinoline | 65442-31-1 | 2 |
| ALLYL CYCLOHEXYL PROP | Allyl cyclohexanepropionate | 2705-87-5 | 1 |
| AMBROXAN | Dodecahydro-3a,6,6,9a-tetramethylnaphtho-(2,1-b)-furane | 3738-00-9 | 1 |
| AMBRETONE | 5-cyclohexadecen-1-one | 37609-25-9 | 1 |
| HEXENYL ACETATE, CIS-3 | | 3681-71-8 | 0.5 |
| ETHYL 2-METHYLBUTYRATE | | 7452-79-1 | 0.5 |

The perfume composition of example 9 can be used in devices for the diffusion of ambiance-generating perfumes, which are available from commercial sources, such as those known under the trade names "Glade ® Plug-In", "Glade ® Wisp", "Reckitt-Benckiser Air-Wick ® Mobil", "Air-Wick ®", "Ambi-Pur Car ®" and "Sara Lee Inspira ®". This composition can advantageously be added to these devices in a volatile solvent (ester, ether etc.), such as for example a glycol ether solvent of the "Dowanol" range (for example Dowanol DPNB or Dowanol DPM - Dow Chemical) in order to modulate the number of days of evaporation.

The perfume compositions can also be added to these devices in the form of a gel obtained by mixing the said perfume composition as illustrated in example 10.

### EXAMPLE 10 : Perfume composition in the form of a gel for a membrane air care device or an electrically powered air care device.

40 % of the perfume composition of example 9 were mixed with 55.97 % of Dowanol DPM, 4 % of the hydrophilic silica known under the trade name "Cab-O-Sil"® (Cabot GmbH) and 0.03 % of Tween 20.

### EXAMPLE 11 : Perfuming oil for candle.

A perfuming oil for a candle was prepared by mixing the ingredients shown in the table below.

### EXAMPLE 12 : Perfuming candle.

A perfuming candle was prepared by melting the wax and the other ingredients in a water bath at 80°C and then adding the perfuming oil according to example 11 and mixing until a uniform mixture was obtained, which was then flowed into a mould with a wick.

The candle thus formed was then left to cool down for 24 hours. This candle was then burnt so as to diffuse the perfume composition in a stable way during at least 24-36 hours.

### EXAMPLE 11 : Perfuming oil for candle.

| Ingredient | Chemical name | CAS N° | Parts per 1000 |
|---|---|---|---|
| HEDIONE | Methyl dihydrojasmonate | 24851-98-7 | 250 |
| LINALOOL | 3,7-dimethyl-1,6-octadien-3-ol | 78-70-6 | 150 |
| MUSK-T | 1,4-Dioxacycloheptadecane-5,17-dione | 105-95-3 | 100 |
| VERTENEX | 4-(1,1-dimethylethyl)cyclohexanol acetate | 32210-23-4 | 100 |
| DI-HYDRO MYRCENOL | 2,6-dimethyl-7-octen-2-ol | 18479-58-8 | 60 |
| VERDOX | o-t-Butylcyclohexyl acetate | 88-41-5 | 50 |
| 1,3,5-TRIMETHOXYBENZENE | | 621-23-8 | 35 |
| CITRAL | 3,7-dimethyl-2,6-octadienal (mixture of cis et trans) | 5392-40-5 | 25 |
| ALLYL CYCLOHEXYL PROP | Allyl cyclohexyl-3 propionate | 2705-87-5 | 20 |
| CITRONELLYL NITRILE, L | (3R)-3,7-dimethyloct-6-enenitrile | 51566-62-2 | 20 |
| LEVOSANDOL | 2-ethyl-4-(2,2,3-trimethyl-3-cyclopenten-1-yl)-2-buten-1-ol | 28219-61-6 | 10 |
| ORBITONE | Octahydro-2,3,8,8-tetramethyl-2-acetonaphthone | 54464-57-2 | 20 |
| AMBROXAN | Dodecahydro-3a,6,6,9a-tetramethylnaphtho (2,1-b) furane | 3738-00-9 | 2 |
| DIMETHYL BENZYL CARBINYL BUTYRATE | | 10094-34-5 | 2 |
| ETHYL 2-METHYLBUTYRATE | | 7452-79-1 | 1 |
| CLONAL | 1-cyanoundecane | 2437-25-4 | 1 |
| ETH VANILLIN | 3-ethoxy-4-hydroxybenzaldehyde | 121-32-4 | 1 |

### EXAMPLE 12 : Perfuming candle.

| NAME | PARTS PER 100 |
|---|---|
| PARAFFIN WAX 52.54 (AIGLON SA) | 22 |
| STEARIC ACID PRISTERENE 9559 (UNIQUIMA) | 20 |
| BEESWAX, CEREWAX A.75 (BAERLOCHER FRANCE) | 8 |
| WHITE VASELINE (AIGLON SA) | 16 |
| LIQUID PARAFFIN (AIGLON SA) | 24 |
| PERFUME COMPOSITION OF EXAMPLE 11 | 10 |

## Claims

1. Use of an alkoxybenzene of formula (I): 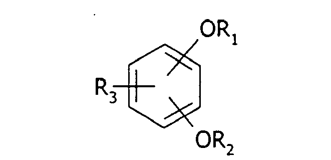 in which :
• R₁ and R₂, which can be the same or different, are a (C₁-C₄) alkyl group, preferably methyl or ethyl;
• R₃ is a (C₁-C₄) alkyl group or a group of formula -OR₄, R₄ being a (C₁-C₄) alkyl group, or of a mixture of alkoxybenzenes of formula (I);
as an odoriferous agent for household products.

2. Use according to claim 1, **characterized in that** the amount of alkoxybenzene is comprised between 0.0001 and 0.1 % by weight of the household product other than for an air care product.

3. Use according to claim 1, **characterized in that** the amount of alkoxybenzene is comprised between 0.01 and 15 % by weight of the air care product.

4. Use according to any of claims 1 to 3, **characterized in that** the alkoxybenzene is chosen among 1,3,5-trimethoxybenzene, 1,3-dimethoxy-5-methylbenzene or mixtures thereof.

## Patentansprüche

1. Verwendung eines Alkylbenzols der Formel (1): 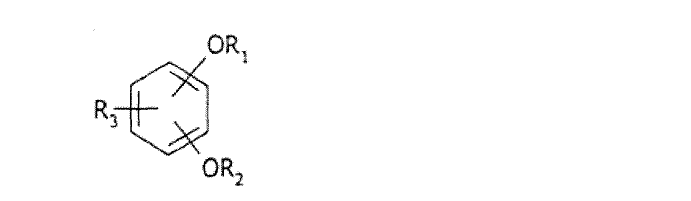 in welcher:
- R₁ und R₂, welche gleich oder verschieden sein können, eine (C₁-C₄)-Alkylgruppe, vorzugsweise Methyl oder Ethyl sind,
- R₃ eine (C₁-C₄)-Alkylgruppe oder eine Gruppe der Formel-OR₄, wobei R₄ eine (C₁-C₄)-Alkylgruppe ist, oder ein Gemisch von Alkylbenzolen mit Formel (l) ist,
als Duftmittel für Haushaltsprodukte.

2. Verwendung nach Anspruch 1, **dadurch gekennzeichnet, daß** die Menge an Alkylbenzol zwischen 0,0001 und 0,1 Gewichtsprozent des Haushaltsproduktes, das kein Luftverbesserungsprodukt ist, liegt.

3. Verwendung nach Anspruch 1, **dadurch gekennzeichnet, daß** die Menge an Alkylbenzol zwischen 0,01 und 15 Gewichtsprozent des Luftverbesserungsprodukts ist.

4. Verwendung nach einem der Ansprüche 1 bis 3, **dadurch gekennzeichnet, daß** das Alkylbenzol gewählt ist unter 1,3,5-Trimethoxybenzol, 1,3-Dimethoxy-5-Methylbenzol oder Gemischen davon.

## Revendications

1. Utilisation d'un alcoxybenzène de formule (I) : 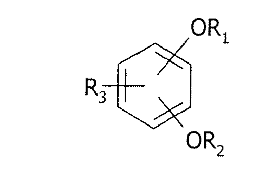 dans laquelle :
• R₁ et R₂, identiques ou différents, représentent un groupe (C₁-C₄)alkyle, de préférence, méthyle ou éthyle ;
• R₃ représente un groupe (C₁-C₄)alkyle ou un groupe de formule -OR₄, R₄ étant un groupe (C₁-C₄)alkyle ou d'un mélange d'alcoxybenzène de formule (I) ;
comme agent odoriférant pour les produits ménagers.

2. Utilisation selon la revendication 1, **caractérisée en ce que** la quantité d'alcoxybenzène est comprise entre 0,0001 et 0,1 % en poids du produit ménager autre qu'un désodorisant d'intérieur.

3. Utilisation selon la revendication 1, **caractérisée en ce que** la quantité d'alcoxybenzène est comprise entre 0,01 et 15 % en poids du désodorisant d'intérieur.

4. Utilisation selon l'une des revendications 1 à 3, **caractérisée en ce que** l'alcoxybenzène est choisi parmi le 1,3,5-triméthoxybenzène, le 1,3-diméthoxy-5-méthylbenzène ou leurs mélanges.
